(19) Europäisches Patentamt
European Patent Office
Office européen des brevets

(11) **EP 2 452 690 A1**

(12) **EUROPEAN PATENT APPLICATION**
published in accordance with Art. 153(4) EPC

(43) Date of publication:
**16.05.2012 Bulletin 2012/20**

(51) Int Cl.:
*A61K 36/73* (2006.01)        *A61P 3/10* (2006.01)
*A61P 9/10* (2006.01)        *A61P 19/00* (2006.01)
*A61P 19/10* (2006.01)        *A61P 25/28* (2006.01)
*A61P 43/00* (2006.01)

(21) Application number: **10797045.1**

(22) Date of filing: **29.06.2010**

(86) International application number:
**PCT/JP2010/061063**

(87) International publication number:
**WO 2011/004734 (13.01.2011 Gazette 2011/02)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **08.07.2009 JP 2009162189**

(71) Applicant: **Arkray, Inc.
Minami-ku
Kyoto-shi
Kyoto 601-8045 (JP)**

(72) Inventors:
• **YAGI, Masayuki.
Kyoto 602-0008 (JP)**
• **IJIMA, Katsumasa.
Tokyo 120-8601 (JP)**

(74) Representative: **Jones, Elizabeth Louise
Dehns
St Bride's House
10 Salisbury Square
GB-London EC4Y 8JD (GB)**

(54) **CARBOXYMETHYLARGININE PRODUCTION INHIBITOR AND COLLAGEN DENATURATION INHIBITOR**

(57) The present invention provides a carboxymethylarginine production inhibitor for inhibiting the production of carboxymethylarginine and a collagen denaturation inhibitor for inhibiting the denaturation of collagen, both of which provide excellent safety. The carboxymethylarginine production inhibitor and the collagen denaturation inhibitor according to the present invention contain an extract of at least one plant selected from the group consisting of plants of *Compositae Anthemis, Sauruaceae Houttuynia, Rosaceae Crataegus,* and *Vitaceae Vitis.* Specifically, they preferably contain an extract of at least one plant selected from the group consisting of *Anthemis nobilis L., Houttuynia cordata Thunberg, Crataegus oxyacantha L.,* and *Vitis vinifera L.*

FIG. 1A
FIG. 1B
FIG. 1C
FIG. 1D
FIG. 1E

## Description

Technical Field

The present invention relates to a carboxymethylarginine production inhibitor and a collagen denaturation inhibitor.

Background Art

Carboxymethylarginine ($N^{\omega}$-carboxymethylarginine, hereinafter referred to as "CMA") is one type of AGE (advanced glycation end-products) specifically produced in collagen. The amount of CMA produced is about 100 times that of pentosidine, which is another type of AGE (Non-Patent Document 1), and it is known that the CMA concentration in the blood of diabetics is higher than that in the blood of nondiabetics (Non-Patent Document 2). Thus, it has been reported that CMA is used as a diagnostic marker for determining or predicting the decrease in elasticity of in *vivo* connective tissues, the development of diabetes complications in connective tissues, damage due to oxidative stress, the progress and the stage of senescence of tissues containing a large amount of collagen, such as skin and bone, and also as a marker for evaluating the drug efficacy of a prophylactic agent, a therapeutic agent, etc. of diseases caused thereby (Patent Document 1).

As a CMA production inhibitor for inhibiting the production of CMA, rutin, quercetin, astragalin, and the like are reported (Patent Document 2). CMA is produced through a reaction between glyoxal as a carbonyl compound and collagen, and the glyoxal is produced through a glycation reaction of a protein and an oxidation reaction, such as the autoxidation, of glucose (Non-Patent Document 3).

Citation List

[Patent Documents]

[Patent Document 1] Japanese Patent No. 3889190
[Patent Document 2] JP 2009-96731 A

[Non-Patent Documents]

[Non-Patent Document 1] Iijima et al., Biochem. J., 2000, Vol. 347, pp. 23-27
[Non-Patent Document 2] Odani et al., Biochem. Biophys. Res. Commun., 2001, Vol. 285, pp. 1232-1236
[Non-Patent Document 3] Thornalley, Ann. N.Y. Acad. Sci., 2005, Vol. 1043, pp. 111-117

Brief Summary of the Invention

Problem to be Solved by the Invention

Thus, in order to prevent the production of CMA in a living organism, it is necessary to inhibit at least one of the reaction (oxidation reaction) between the glyoxal and collagen and the glycation reaction of a protein.

An object of the present invention is to provide a novel CMA production inhibitor for inhibiting the production of CMA and a novel collagen denaturation inhibitor for inhibiting the denaturation of collagen, both of which provide excellent safety.

Means for Solving Problem

The present invention provides a CMA production inhibitor containing: an extract of at least one plant selected from the group consisting of plants of *Compositae Anthemis, Saururaceae Houttuynia, Rosaceae Crataegus,* and *Vitaceae Vitis.*

The present invention also provides a collagen denaturation inhibitor containing: an extract of at least one plant selected from the group consisting of plants of *Compositae Anthemis, Saururaceae Houttuynia, Rosaceae Crataegus,* and *Vitaceae Vitis.*

The present invention also provides a method for inhibiting production of CMA, including: adding the CMA production inhibitor of the present invention to a sample containing collagen.

The present invention also provides a method for inhibiting denaturation of collagen, including: adding the collagen denaturation inhibitor of the present invention to a sample containing collagen.

The present invention also provides a method for treating damage due to disease, senescence and the hypofunction of tissue, and oxidative stress caused by the production of CMA, including: administering the CMA production inhibitor of the present invention to a living organism.

The present invention also provides a method for treating damage due to disease, senescence and the hypofunction of tissue, and oxidative stress caused by denaturation of collagen, including: administering the collagen denaturation inhibitor of the present invention to a living organism.

Effects of the Invention

According to the present invention, by containing the above-described extract(s), it is possible to inhibit the progress of a reaction in at least one of the following reaction pathways included in the CMA-producing pathway: the reaction pathway of glycation of a protein and the reaction pathway of oxidation of collagen by glyoxal. Thus, the present invention can inhibit the production of CMA in *vivo* and the denaturation of collagen, for example. Therefore, the present invention can be used in a method for treating or preventing damage and the like due to disease, senescence and the hypofunction of tissue, and oxidative stress caused by the production of CMA *in vivo* or the denaturation of collagen. Furthermore, by adding the inhibitor of the present invention to a sample containing collagen, it is possible to prevent the deterioration of the quality of the sample caused by the production of CMA or the denaturation of collagen. Furthermore, the present invention provides excellent safety because it uses plant extracts that have been used since ancient times. Also, the present invention provides excellent productivity because the plant extracts can be obtained in large quantities.

Brief Description of Drawings

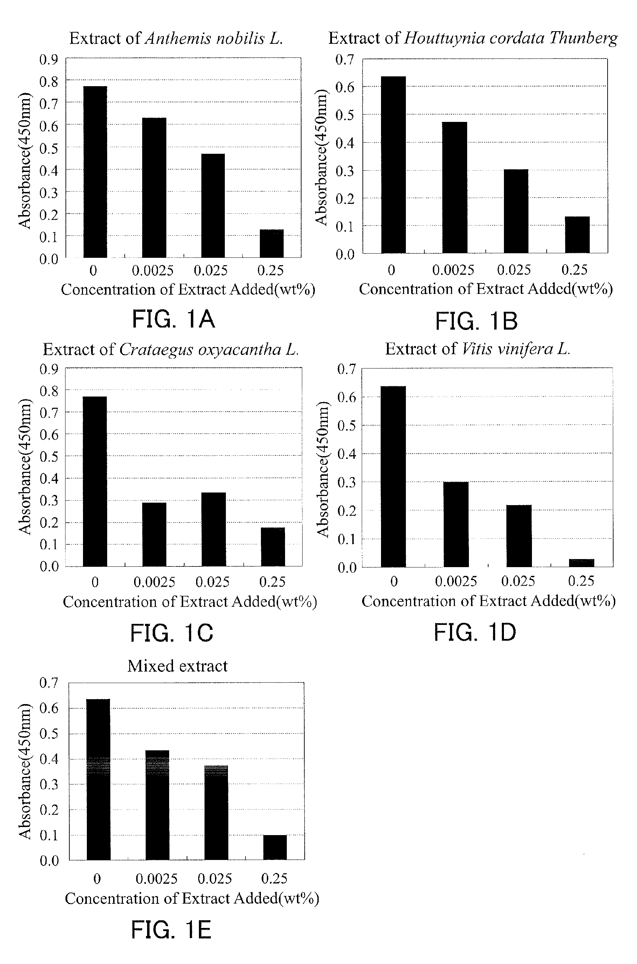

[FIG. 1]FIGs. 1A to 1E are graphs showing the measurement results indicating the effect of inhibiting CMA production through the above-described glycation reaction by respective plant extracts and a mixed extract. FIG. 1A shows the measurement results obtained when the extract of *Anthemis nobilis L.* was used. FIG. 1B shows the measurement results obtained when the extract of *Houttuynia cordata Thunberg* was used. FIG. 1C shows the measurement results obtained when the extract of *Crataegus oxyacantha L.* was used. FIG. 1D shows the measurement results obtained when the extract of *Vitis vinifera L.* was used. FIG. 1E shows the measurement results obtained when the mixed extract was used.

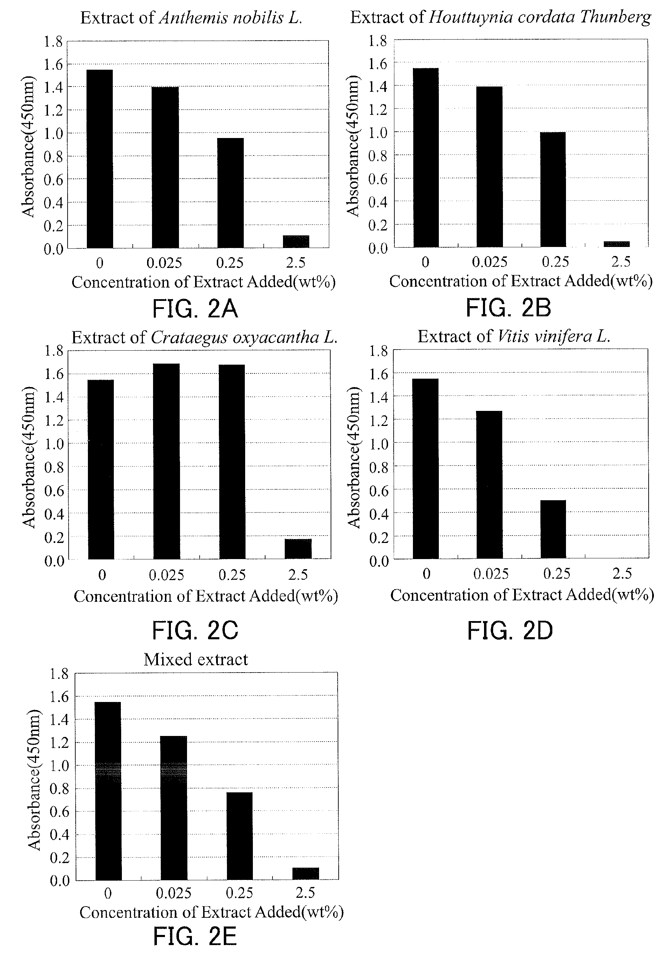

[FIG. 2] FIGs. 2A to 2E are graphs showing the measurement results indicating the effect of inhibiting CMA production through the above-described oxidation reaction by the respective plant extracts and the mixed extract. FIG. 2A shows the measurement results obtained when the extract of *Anthemis nobilis L.* was used. FIG. 2B shows the measurement results obtained when the extract of *Houttuynia cordata Thunberg* was used. FIG. 2C shows the measurement results obtained when the extract of *Crataegus oxyacantha L.* was used. FIG. 2D shows the measurement results obtained when the extract of *Vitis vinifera L.* was used. FIG. 2E shows the measurement results obtained when the mixed extract was used.

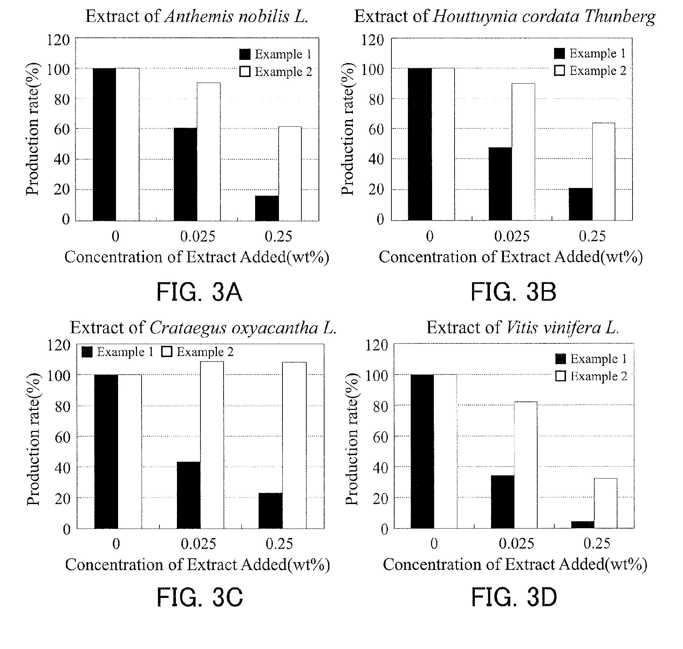

[FIG. 3] FIGs. 3A to 3D are graphs showing the CMA production rate in reaction solutions to which the respective plant extracts were added. FIG. 3A shows the CMA production rate when the extract of *Anthemis nobilis L.* was used. FIG. 3B shows the CMA production rate when the extract of *Houttuynia cordata Thunberg* was used. FIG. 3C shows the CMA production rate when the extract of *Crataegus oxyacantha L.* was used. FIG. 3D shows the CMA production rate when the extract of *Vitis vinifera L.* was used.

Mode for Carrying Out the Invention

The CMA production inhibitor of the present invention preferably contains: an extract of a plant of *Compositae Anthemis*; an extract of a plant of *Saururaceae Houttuynia*; an extract of a plant of *Rosaceae Crataegus*; and an extract of a plant of *Vitaceae Vitis,* for example.

In the CMA production inhibitor of the present invention, it is preferable that the plant of *Compositae Anthemis* is *Anthemis nobilis L.,* the plant of *Saururaceae Houttuynia* is *Houttuynia cordata Thunberg,* the plant of *Rosaceae Crataegus* is *Crataegus oxyacantha L.,* and the plant of *Vitaceae Vitis* is *Vitis vinifera L.,* for example.

The collagen denaturation inhibitor of the present invention preferably contains: an extract of a plant of *Compositae Anthemis*; an extract of a plant of *Saururaceae Houttuynia*; an extract of a plant of *Rosaceae Crataegus*; and

an extract of a plant of *Vitaceae Vitis,* for example.

In the collagen denaturation inhibitor of the present invention, it is preferable that the plant of *Compositae Anthemis* is *Anthemis nobilis L.,* the plant of *Saururaceae Houttuynia* is *Houttuynia cordata Thunberg,* the plant of *Rosaceae Crataegus* is *Crataegus oxyacantha L.,* and the plant of *Vitaceae Vitis* is *Vitis vinifera L.,* for example.

The present invention will be described in detail below.

As described above, the CMA production inhibitor of the present invention can inhibit at least one of the following reaction pathways included in the CMA-producing pathway: the reaction pathway of glycation of a protein and the reaction pathway of oxidation of collagen by glyoxal. For example, the CMA production inhibitor of the present invention preferably can inhibit the reaction pathway of oxidation of collagen by glyoxal, more preferably both the reaction pathways.

The CMA production inhibitor of the present invention contains an extract of at least one plant selected from the group consisting of plants of *Compositae Anthemis, Saururaceae Houttuynia, Rosaceae Crataegus,* and *Vitaceae Vitis,* as described above. The CMA production inhibitor may contain one kind of extract or two or more kinds of extracts obtained from the above-described plants, for example.

The CMA production inhibitor preferably contains an extract of a plant of *Vitaceae Vitis* and an extract of a plant of *Rosaceae Crataegus,* for example. Also, the CMA production inhibitor preferably contains an extract of a plant of *Compositae Anthemis,* an extract of a plant of *Saururaceae Houttuynia,* an extract of a plant of *Rosaceae Crataegus,* and an extract of a plant of *Vitaceae Vitis,* for example.

When the CMA production inhibitor contains an extract (A) of a plant of *Compositae Anthemis,* an extract (B) of a plant of *Saururaceae Houttuynia,* an extract (C) of a plant of *Rosaceae Crataegus,* and an extract (D) of a plant of *Vitaceae Vitis,* the blending ratio of these extracts (the dry weight ratio, A : B : C : D) is not particularly limited, and preferably is 1 : 0.01 to 100 : 0.01 to 100 : 0.01 to 100, for example.

Examples of the plant of *Compositae Anthemis* (*Chamaemelum*) include *Anthemis nobilis L.* (= *Chamaemelum nobile*). An extract of the plant of *Compositae Anthemis* may be obtained from its flower, spike, pericarp, fruit, stem, leaf, rhizome, root bark, root, and seed, for example. The extract may be obtained from one part or from at least two parts. Alternatively, the extract may be obtained from the entire plant. In the extract of the plant of *Compositae Anthemis,* the part from which the extract is obtained is not particularly limited. The extract may be an extract of anthodia, for example.

Examples of the plant of *Saururaceae Houttuynia* include *Houttuynia cordata Thunberg.* An extract of the plant of *Saururaceae Houttuynia* may be obtained from its flower, spike, pericarp, fruit, stem, leaf, rhizome, root bark, root, and seed, for example. The extract may be obtained from one part or from at least two parts. Alternatively, the extract may be obtained from the entire plant. In the extract of the plant of *Saururaceae Houttuynia,* the part from which the extract is obtained is not particularly limited. The extract may be an extract of aerial parts including the flower, spike, pericarp, fruit, stem, leaf, branch, branches and leaves, trunk, bark, seed, and the like, for example.

Examples of the plant of *Rosaceae Crataegus* include *Crataegus oxyacantha L.* and *C. cuneata Sieb. et Zucc.* An extract of the plant of *Rosaceae Crataegus* may be obtained from its flower, spike, pericarp, fruit, stem, leaf, branch, branches and leaves, trunk, bark, rhizome, root bark, root, and seed, for example. The extract may be obtained from one part or from at least two parts. Alternatively, the extract may be obtained from the entire plant. In the extract of the plant of *Rosaceae Crataegus,* the part from which the extract is obtained is not particularly limited. The extract may be an extract of the fruit, for example.

Examples of the plant of *Vitaceae Vitis* include: *Vitis vinifera L.*; *Vitis labrusca L.; V. saccharifera Makino*; *V. ficifolia Bunge var. lobata (Regel) Nakai*; *V. flexuosa Thunb.*; *V. coiguetiae Pulliat*; and *V. labruscana Bailey.* An extract of the plant of *Vitaceae Vitis* may be obtained from its flower, spike, pericarp, fruit, stem, leaf, branch, branches and leaves, trunk, bark, rhizome, root bark, root, and seed, for example. The extract may be obtained from one part or from at least two parts. Alternatively, the extract may be obtained from the entire plant. In the extract of the plant of *Vitaceae Vitis,* the part from which the extract is obtained is not particularly limited. The extract may be an extract of the leaves, for example.

The CMA production inhibitor of the present invention preferably contains an extract of at least one plant selected from the group consisting of *Anthemis nobilis L., Houttuynia cordata Thunberg, Crataegus oxyacantha L.,* and *Vitis vinifera* L., for example. The CMA production inhibitor may contain an extract of one of the above-described plants, or it may contain extracts of two or more of the above-described plants, for example. The CMA production inhibitor preferably contains an extract of *Vitis vinifera L.* and an extract of *Crataegus oxyacantha L.,* for example. Also, the CMA production inhibitor preferably contains an extract of *Anthemis nobilis L.,* an extract of *Houttuynia cordata Thunberg,* an extract of *Crataegus oxyacantha L.,* and an extract of *Vitis vinifera L.,* for example.

When the CMA production inhibitor contains an extract (a) of *Anthemis nobilis L.,* an extract (b) of *Houttuynia cordata Thunberg,* an extract (c) of *Crataegus oxyacantha L.,* and an extract (d) of *Vitis vinifera L.,* the blending ratio of these extracts (the dry weight ratio, a : b : c : d) is not particularly limited, and preferably is 1 : 0.01 to 100 : 0.01 to 100 : 0.01 to 100, for example.

The CMA production inhibitor of the present invention preferably contains at least one extract selected from the group consisting of an extract obtained from the anthodia of a plant of *Compositae Anthemis,* an extract obtained from the aerial parts of a plant of *Saururaceae Houttuynia,* an extract obtained from the fruits of a plant of *Rosaceae Crataegus,* and an extract obtained from the leaves of a plant of *Vitaceae Vitis,* for example. The CMA production inhibitor may contain one kind, two or more kinds, or all kinds of the above-described extracts, for example. Specifically, the CMA production inhibitor preferably contains, for example, at least one extract selected from the group consisting of an extract obtained from the anthodia of *Anthemis nobilis L.,* an extract obtained from the aerial parts of *Houttuynia cordata Thunberg,* an extract obtained from the fruits of *Crataegus oxyacantha L.,* and an extract obtained from the leaves of *Vitis vinifera L.* The CMA production inhibitor may contain one kind, two or more kinds, or all kinds of the above-described extracts, for example.

In the present invention, the extract can be obtained from any of the above-described parts or from the entire plant, for example. The method for obtaining the extract is not particularly limited. Examples thereof include known methods such as solvent extraction and expression.

An extraction solvent to be used in the solvent extraction is not particularly limited. Examples thereof include aqueous solvents and organic solvents. The aqueous solvents are not particularly limited. Examples thereof include water. The organic solvents are not particularly limited. Examples thereof include lower alcohols, polyhydric alcohols, ketones, esters, ethers, nitriles, aromatic compounds, and alkyl chlorides. The lower alcohols are not particularly limited. Examples thereof include methanol, ethanol, and absolute ethanol. The polyhydric alcohols are not particularly limited. Examples thereof include propylene glycol and 1,3-butylene glycol. The ketones are not particularly limited. Examples thereof include acetone and formic acid. The esters are not particularly limited. Examples thereof include ethyl acetate. The ethers are not particularly limited. Examples thereof include diethyl ether and dioxane. The nitriles are not particularly limited. Examples thereof include acetonitrile. The aromatic compounds are not particularly limited. Examples thereof include benzene, toluene, and xylene. The alkyl chlorides are not particularly limited. Examples thereof include chloroform.

As the above-described extraction solvent, one kind of solvent may be used, or two or more kinds of solvents may be used in combination, for example. Furthermore, the extraction solvent may be a mixed solvent of the aqueous solvent and the organic solvent, for example. The mixed solvent is not particularly limited. Examples thereof include lower alcohol aqueous solutions such as ethanol aqueous solutions. The proportion of the organic solvent in the mixed solvent is not particularly limited, and is, for example, 1 to 99 vol%.

The solvent extraction can be carried out by, for example, using any of the above-described parts or the entire plant as the raw material and immersing it in the extraction solvent. The raw material may be subjected to treatments such as, for example, washing, drying, and pulverizing, prior to the immersion. When there are two or more kinds of raw materials, each of the raw materials may be subjected to an extraction treatment separately, or a mixture of the two or more kinds of raw materials may be subjected to an extraction treatment. In the former case, for example, the resultant extracts may be mixed together to provide a mixed extract. The proportions of the respective extracts in the mixed extract are not particularly limited, and may be equal proportions (weights), for example. For example, in the case where the above-described four kinds of plants are used, a mixed extract preferably is prepared by mixing extracts of these plants at a ratio (weight ratio) of 1 : 1 : 1 : 1. In the latter case, for example, the proportions of the respective raw materials in the mixture are not particularly limited, and may be equal proportions (weights), for example. For example, when the above-described four kinds of plants are used, it is preferable to perform an extraction treatment with respect to a mixture obtained by mixing the respective raw materials at a ratio (weight ratio) of 1 : 1 : 1 : 1.

The ratio between the raw material used in the extraction and the extraction solvent is not particularly limited, and may be, for example, 100 g (dry weight) of the raw material and 1 to 1000 l of the extraction solvent, preferably 1 to 100 l of the extraction solvent. The immersion time can be set as appropriate depending on, for example, the kinds, the amounts, and the like of the raw material and the extraction solvent, and is not particularly limited. Specifically, in the case where 100 g of the raw material is immersed in 10 l of the extraction solvent, the immersion time preferably is 0.5 hours or more, more preferably 0.5 to 24 hours, for example.

The temperature of the extraction solvent at the time of extraction may be, for example, room temperature, room temperature or higher, or room temperature or lower, and is not particularly limited. When the extraction solvent is an aqueous solvent, the extraction treatment preferably is hot water extraction, for example. In the case of the hot water extraction, the temperature of the aqueous solvent is not particularly limited, and is, for example, 30°C or higher, preferably 50°C to 100°C. Furthermore, the treatment time in the hot water extraction can be set as appropriate depending on, for example, the kind and the amount of the raw material, the amount of the aqueous solvent, and the like, and is not particularly limited. Specifically, when extraction from 100 g (dry weight) of the raw material is performed using 10 l of the aqueous solvent, the treatment time preferably is 0.5 hours or longer, more preferably 0.5 to 24 hours, for example.

After the extraction treatment, the extract obtained may be subjected to a purification treatment, for example. The purification treatment is not particularly limited. Examples thereof include known methods such as a distillation treatment, a filtration treatment, a chromatography treatment, and a drying treatment.

The form of the extract is not particularly limited. For example, the extract may be in the form of liquid, paste, powder, or the like. The form of the extract can be selected as appropriate depending on the form of the inhibitor described below.

The CMA production inhibitor may contain only the above-described extract, or it may be a composition containing the above-described extract and other components. Examples of the components include various kinds of additives such as an excipient, a binding agent, a lubricant, a disintegrant, an absorption promoter, an emulsifying agent, a stabilizing agent, and an antiseptic agent. The blended amounts of the various kinds of additives in the CMA production inhibitor are not particularly limited, and can be set as appropriate.

The form of the CMA production inhibitor is not particularly limited. For example, the CMA production inhibitor may be in the form of solid, gel, liquid, or the like. Specific examples of the form of the CMA production inhibitor include powder, microgranule, granule, tablet, coated tablet, capsule, troche, eye drop, liquid, patch, lotion, and ointment.

The production of CMA in *vivo* can be inhibited by administering the CMA production inhibitor of the present invention to a living organism, for example. Thus, the CMA production inhibitor of the present invention can be used in, for example, a method for treating or preventing damage and the like due to disease, senescence and the hypofunction of tissue, and oxidative stress caused by the production of CMA in *vivo,* as will be described below. The CMA production inhibitor may be administered in the form of a composition obtained by mixing it with components of another article, e.g., foods such as common foods and nutritional supplements, cosmetics, quasi drugs, and pharmaceuticals, for example. The blended amount of the CMA production inhibitor in the composition can be set as appropriate depending on, for example, the kind of article, subjects to which the composition is administered, and the like, and is not particularly limited.

Next, the collagen denaturation inhibitor of the present invention may contain an extract of at least one plant selected from the group consisting of plants of *Compositae Anthemis, Saururaceae Houttuynia, Rosaceae Crataegus,* and *Vitaceae Vitis,* as described above.

The composition of the collagen denaturation inhibitor, the respective extracts contained therein, and the extraction method for extracting them are the same as those described above for the CMA production inhibitor. Furthermore, various kinds of additives contained in the collagen denaturation inhibitor other than the extracts, the form of the collagen denaturation inhibitor, and the like are the same as those described above for the CMA production inhibitor.

The denaturation of collagen in *vivo* may be inhibited by administering the collagen denaturation inhibitor of the present invention to a living organism, for example. Thus, the collagen denaturation inhibitor of the present invention may be used in, for example, a method for treating or preventing damage and the like due to disease, senescence and the hypofunction of tissue, and oxidative stress caused by the denaturation of collagen in *vivo,* as will be described below. The collagen denaturation inhibitor may be administered in the form of a composition obtained by mixing it with components of another article, e.g., foods such as common foods and nutritional supplements, cosmetics, quasi drugs, and pharmaceuticals, for example. The blended amount of the collagen denaturation inhibitor in the composition can be set as appropriate depending on, for example, the kind of article, subjects to which the composition is administered, and the like, and is not particularly limited.

The method for inhibiting the production of CMA according to the present invention is a method for inhibiting the production of CMA in collagen, including: adding the CMA production inhibitor of the present invention to a sample containing collagen, as described above.

The sample containing collagen is not particularly limited. Examples thereof include industrial products and foods containing collagen. The industrial products are not particularly limited. Examples thereof include collagen extracts, metal-leaching solutions for refining nonferrous metals, adhesives, adhesives for musical instruments, vinyl polymers, paints, films, gelatin films, microcapsules, ready-mixed concrete, artificial leather, sandpaper, heat insulating materials for cold weather regions, photographic paper, ignitable substances coating a match head, and sumi (Indian ink). The foods are not particularly limited. Examples thereof include: meat such as fibrous beef, wing tip, chicken skin, gristle, and pettitoes; fish such as pufferfish, anglerfish, abalone, soft-shelled turtles, sea cucumber, loach, and sharks' fin; processed foods of the above-described meat and fish; collagen processed foods such as collagen drinks and collagen-containing confectionery and snacks; and collagen-containing nutritional supplements.

The method for adding the CMA production inhibitor to the sample containing collagen is not particularly limited. Any conventionally known method can be employed depending on the kind of sample, for example. When the sample containing collagen is an industrial product or a food like the above-described processed food, collagen processed food, or collagen-containing nutritional supplement, the CMA production inhibitor may be blended in the sample containing collagen, for example. The amount of the CMA production inhibitor blended in the sample containing collagen can be set as appropriate depending on the sample containing collagen, and is not particularly limited. When the sample containing collagen is a food like the above-described meat and fish, a treatment solution containing the CMA production inhibitor may be applied to or sprayed onto the sample containing collagen, or the sample may be immersed in the treatment solution, for example. The composition of the treatment solution is not particularly limited. Examples include water and a pH adjuster. The concentration of the CMA production inhibitor in the treatment solution is not particularly limited, and is, for example, 0.001 to 99.9 wt%, preferably 0.001 to 10.0 wt%.

The method for inhibiting the denaturation of collagen according to the present invention includes: adding the collagen denaturation inhibitor of the present invention to a sample containing collagen, as described above. In the method for inhibiting the denaturation of collagen, the sample containing collagen is not particularly limited. Examples thereof include the above-described samples containing collagen. Furthermore, the method for adding the collagen denaturation inhibitor to the sample containing collagen also is not particularly limited, and the above description of the method for adding the CMA production inhibitor also is applied thereto, for example.

The present invention provides a method for treating damage due to disease, senescence and the hypofunction of tissue, and/or oxidative stress caused by the production of CMA, including: administering the CMA production inhibitor of the present invention to a living organism. The treatment method of the present invention may be a prevention method for preventing damage due to disease, senescence and the hypofunction of tissue, and/or oxidative stress caused by the production of CMA, and includes: administering the CMA production inhibitor of the present invention to a living organism. Examples of the disease include diabetes complications, Alzheimer's disease, arteriosclerosis, osteoporosis, scleroderma, rheumatism, and osteoarthropathy. Examples of the tissue include those containing collagen. Specific examples of such tissues include skin, bone, blood vessels, joints, tendons, organs, and brain tissues. The animal species of the living organism is not particularly limited. Examples thereof include: humans; nonhuman mammals such as monkeys, cows, pigs, dogs, and cats; birds such as chickens; and fish and shellfish. The administration method is not particularly limited. Examples thereof include oral administration and parenteral administration. Examples of parenteral administration include transdermal absorption and injections such as intravenous injection. The form of the CMA production inhibitor is not particularly limited, and may be in any of the above-described forms, for example. Furthermore, the CMA production inhibitor may be administered in the form of a composition obtained by mixing it with other components, as described above. The blended amount of the CMA production inhibitor in the composition can be set as appropriate, and is not particularly limited, as described above. The dose of the CMA production inhibitor can be set as appropriate depending on, for example, the animal species and the age of the animal, and is not particularly limited. When a healthy adult takes the CMA production inhibitor orally, it is preferable that 10 to 2000 mg, more preferably 50 to 1000 mg of the extract(s) (solid content) is taken, for example. The CMA production inhibitor of the present invention also can be referred to as a therapeutic agent for treating damage due to disease, senescence and the hypofunction of tissue, and/or oxidative stress, and it also encompasses a prophylactic agent, for example.

The present invention also provides a method for treating damage due to disease, senescence and the hypofunction of tissue, and oxidative stress caused by the denaturation of collagen, including: administering the collagen denaturation inhibitor of the present invention to a living organism. The treatment method of the present invention may be a prevention method for preventing damage due to disease, senescence and the hypofunction of tissue, and oxidative stress caused by denaturation of collagen, and includes: administering the collagen denaturation inhibitor of the present invention to a living organism. In the treatment method that includes administering the collagen denaturation inhibitor, the disease, the tissue, the animal species of the living organism, the administration method, the administration form, the blended amount, and the dose are the same as those described above for the treatment method that includes administering the CMA production inhibitor, for example. The collagen denaturation inhibitor of the present invention also can be referred to as a therapeutic agent for treating damage due to disease, senescence and the hypofunction of tissue, and/or oxidative stress, and also encompasses a prophylactic agent, for example.

An extract of the present invention includes an extract of a plant of *Compositae Anthemis, Saururaceae Houttuynia, Rosaceae Crataegus* or *Vitaceae Vitis,* for use in the treatment of damage due to disease, senescence and the hypofunction of tissue, and/or oxidative stress caused by the production of CMA or the denaturation of collagen. The treatment of damage may be prevention of damage. A composition of the present invention is a composition containing an extract of at least one plant selected from the group consisting of plants of *Compositae Anthemis, Saururaceae Houttuynia, Rosaceae Crataegus,* and *Vitaceae Vitis,* for use in the treatment of damage due to disease, senescence and the hypofunction of tissue, and/or oxidative stress caused by the production of CMA or the denaturation of collagen. The treatment of damage may be prevention of damage. In the extract and the composition of the present invention, the extract of each kind and the composition containing the extract(s) are as described above, and the combination, the method for using them, and the like also are as described above.

Examples

Next, examples of the present invention will be described. It is to be noted, however, the present invention is by no means limited by the following examples.

(Example 1)

In the present example, inhibition of the production of CMA by inhibiting the progress of a reaction in a reaction pathway of glycation of a protein (collagen) was evaluated. Specifically, the evaluation was carried out by using collagen and glucose as starting materials to produce CMA and measuring the inhibition of the production of CMA by plant extracts and a mixed extract. Plants used in the present examples were: anthodia of *Anthemis nobilis L.*; aerial parts of *Houttuynia cordata Thunberg*; fruit of *Crataegus oxyacantha L.*; and leaves of *Vitis vinifera L.*

<u>Preparation of Plant Extract</u>

100 g of each of the plants in the dried state were immersed in 101 of purified water at 80°C for about 5 hours, from which a liquid extract of each plant was obtained. Each liquid extract was filtered to remove the residue, and about 10 kg of the filtrate was collected. Each filtrate was further dried to remove the solvent. Thus, extracts of the respective plants in the form of powder were obtained. Furthermore, as the mixed extract, "AG Herb MIX" (trade name, ARRAY, Inc.) was used. The plant extracts and the mixed extract were used as samples.

<u>Evaluation by ELISA</u>

An anti-CMA antibody solution (IgG concentration: 1 $\mu$g/ml) was prepared in the same manner as described in Example 1 of JP 2002-243732 A, except that the immunized animal was changed to a goat. Collagen-glucose solutions containing the respective samples at predetermined concentrations (0, 0.0025, 0.025, and 0.25 wt%) and having the following composition were also prepared. Each of the collagen-glucose solutions was allowed to react at 60°C for 40 hours, thus obtaining a reaction solution. 100 $\mu$l of the reaction solution was added to each well of a 96-well ELISA plate (Nunc Maxisorp) and allowed to stand still at room temperature for 2 hours to immobilize the reaction solution on each well. After the reaction solution was removed, 100 $\mu$l of a blocking agent (N102, NOF CORPORATION) was added to each well and allowed to stand still at room temperature for 1 hour to perform blocking. The blocking agent was then removed, and thereafter, 0.1 ml of the anti-CMA antibody solution was added to each well and allowed to react at room temperature for 1 hour. After the reaction, each well was washed three times with a washing solution containing 0.1 wt% Tween (registered trademark) 20, 0.2 mol/l NaCl, and 50 mmol/l Tris-HCl (pH 7.5). After the washing, 0.1 ml of a HRP-anti-goat antibody (Cappel) was added to each well as a secondary antibody and allowed to react at room temperature for 1 hour. Each well was washed with the washing solution, and thereafter, 0.05 ml of a TMB (3,3'5,5'-tetramethylbenzidine) reagent (Kem-En-Tec) was added to each well, and 0.05 ml of 1 mol/l sulfuric acid was further added to terminate the reaction. Thereafter, the absorbance of the reaction solution contained in each well was measured at a measurement wavelength of 450 nm using a microplate reader. As the absorbance becomes relatively lower, it means that the production of CMA is inhibited more strongly.

(Collagen-glucose solution)

| Component | Concentration |
| --- | --- |
| collagen | 1 mg/ml |
| glucose | 0.2 mol/l |
| sample | predetermined concentration |

The measurement results regarding the effect of inhibiting the production of CMA by the respective plant extracts and the mixed extract are shown in the graphs of FIGs. 1A to 1E. FIG. 1A shows the measurement results obtained when the extract of *Anthemis nobilis L.* was used. FIG. 1B shows the measurement results obtained when the extract of *Houttuynia cordata Thunberg* was used. FIG. 1C shows the measurement results obtained when the extract of *Crataegus oxyacantha L.* was used. FIG. 1D shows the measurement results obtained when the extract of *Vitis vinifera L.* was used. FIG. 1E shows the measurement results obtained when the mixed extract was used. In the graphs of FIGs. 1A to 1E, the horizontal axis indicates the concentration (wt%) of the sample added, and the vertical axis indicates the absorbance at the measurement wavelength of 450 nm. As can be seen from the graphs of FIGs. 1A to 1E, a decrease in the absorbance was observed when each sample was present, so that it was confirmed that each sample exhibited the effect of inhibiting the production of CMA. Furthermore, in every sample, the amount of CMA produced was reduced substantially in keeping with the increase in the amount of the sample added.

Furthermore, assuming that the absorbance of the reaction solution containing no sample (0%) was 100%, the $IC_{50}$ of each sample was determined by calculating the concentration of the sample at which the absorbance became 50%. The thus-determined $IC_{50}$ was, in descending order of magnitude of the effect of inhibiting CMA production: 0.002 wt% in the case of the extract of *Crataegus oxyacantha L.*; 0.003 wt% in the case of the extract of *Vitis vinifera L.*; 0.020 wt% in the case of the extract of *Houttuynia cordata Thunberg*; 0.021 wt% for the mixed extract; and 0.030 wt% in the case of the extract of *Anthemis nobilis L.*

(Example 2)

In the present example, inhibition of the production of CMA by inhibiting the progress of a reaction in the reaction pathway of oxidation of a protein (collagen) by glyoxal was evaluated. Specifically, the evaluation was carried out by using collagen and glyoxal as starting materials to produce CMA and measuring the inhibition of the production of CMA by the plant extracts and the mixed extract. In the present example, sample preparation and evaluation of inhibition of the production of CMA were carried out in the same manner as in Example 1, except that a collagen-glyoxal solution having the following composition was used instead of the collagen-glucose solution and the concentration of the sample contained therein was set to predetermined concentrations (0, 0.025, 0.25, and 2.5 wt%).

(Collagen-glyoxal solution)

| Component | Concentration |
| --- | --- |
| collagen | 1 mg/ml |
| glyoxal | 0.002 mol/l |
| sample | predetermined concentration |

The measurement results regarding the effect of inhibiting the production of CMA by the respective plant extracts and the mixed extract are shown in the graphs of FIGs. 2A to 2E. FIG. 2A shows the measurement results obtained when the extract of *Anthemis nobilis L.* was used. FIG. 2B shows the measurement results obtained when the extract of *Houttuynia cordata Thunberg* was used. FIG. 2C shows the measurement results obtained when the extract of *Crataegus oxyacantha L.* was used. FIG. 2D shows the measurement results obtained when the extract of *Vitis vinifera L.* was used. FIG. 2E shows the measurement results obtained when the mixed extract was used. In the graphs of FIGs. 2A to 2E, the horizontal axis indicates the concentration (wt%) of the sample added, and the vertical axis indicates the absorbance at the measurement wavelength of 450 nm. As can be seen from the graphs of FIGs. 2A to 2E, a decrease in the absorbance was observed when each sample was present, so that it was confirmed that each sample exhibited the effect of inhibiting the production of CMA. Furthermore, in every sample, the amount of CMA produced was reduced substantially in keeping with the increase in the amount of the sample added. Also, the $IC_{50}$ of each sample was determined in the same manner as in Example 1. The thus-determined $IC_{50}$ was, in descending order of magnitude of the effect of inhibiting CMA production: 0.129 wt% in the case of the extract of *Vitis vinifera L.*; 0.191 wt% in the case of the mixed extract; 0.280 wt% in the case of the extract of *Houttuynia cordata Thunberg*; 0.292 wt% in the case of the extract of *Anthemis nobilis L.*; and 0.629 wt% in the case of the extract of *Crataegus oxyacantha L.*

Furthermore, from the measurement results obtained in Examples 1 and 2, the relative ratio of CMA production (CMA production rate, %) of each sample was determined using the following equation.

$$\text{CMA production rate } (\%) = (\text{a/b}) \times 100$$

a: the absorbance of the reaction solution containing each sample  
b: the absorbance of the reaction solution containing no sample (0%)

The CMA production rates (%) of the reaction solutions containing the respective plant extracts are shown in the graphs of FIGs. 3A to 3D. FIG. 3A is a graph showing the CMA production rate when the extract of *Anthemis nobilis L.* was used. FIG. 3B is a graph showing the CMA production rate when the extract of *Houttuynia cordata Thunberg* was used. FIG. 3C is a graph showing the CMA production rate when the extract of *Crataegus oxyacantha L.* was used. FIG. 3D is a graph showing the CMA production rate when the extract of *Vitis vinifera L.* was used. In the graphs of FIGs. 3A to 3D, the horizontal axis indicates the concentration (wt%) of the sample added, the vertical axis indicates the CMA production rate (%), the left bars indicate the measurement results regarding the inhibition of the collagen glycation reaction pathway in Example 1, and the right bars indicate the measurement results regarding the inhibition of the collagen oxidation reaction pathway involving glyoxal in Example 2.

As can be seen from the graphs of FIGs. 3A , 3B, and 3D, when the extracts of *Anthemis nobilis L., Houttuynia cordata Thunberg,* and *Vitis vinifera L.* were used, a decrease in CMA production rate was observed in both Examples 1 and 2 when the sample concentration was 0.025 to 0.25 wt%. This demonstrates that the glycation reaction pathway and the oxidation reaction pathway were both inhibited. On the other hand, as can be seen from the graph of FIG. 3C, in the case of the extract of *Crataegus oxyacantha L.,* the decrease in CMA production rate in Example 1 was much more significant that in Example 2 when the concentration thereof was 0.025 to 0.25 wt%. This demonstrates that the glycation reaction pathway, in particular, was strongly inhibited.

As described above, it was demonstrated that the extract of *Anthemis nobilis L.,* the extract of *Houttuynia cordata Thunberg,* the extract of *Crataegus oxyacantha L.,* the extract of *Vitis vinifera L.,* and the mixed extract inhibit the production of CMA in collagen and the denaturation of collagen. In particular, it was revealed that these extracts inhibit the production of CMA by inhibiting at least one of the glycation reaction pathway and the oxidation reaction pathway.

(Example 3)

In the present example, the influence of the mixed extract evaluated in Examples 1 and 2 on the serum CMA concentration when it was taken was evaluated.

Foods in capsule form, each containing 600 mg of the mixed extract "AG herb MIX" (trade name, ARKRAY, Inc.) used in Example 1, were provided. Subjects were: eight healthy subjects in a healthy subject group and four subjects, including diabetics and hyperglycemia patients, in a high blood glucose group. Each of the subjects took the food in capsule form once a day for 12 weeks without interruption. Immediately before the start of the intake, blood was collected from each subject by an ordinary method to prepare a serum sample before the intake. Also, 12 weeks after the start of the intake, blood was collected from each subject in the same manner to prepare a serum sample after the intake.

Next, according to the measurement method of Odani et al. (Non-Patent Document 2), the CMA concentration in the blood was measured in the following manner using the serum samples before and after the intake. First, 0.2 ml of each serum sample was mixed with 0.8 ml of cold ethanol, and the resultant mixture was cooled for 20 minutes. After the cooling, this mixture was centrifuged at 10,000 rpm for 20 minutes, and the precipitate was collected. 1 ml of a phosphate buffer solution containing 0.2 mg/ml Porcine intestine peptidase (code: P7500, Sigma Chemical Co.) was added to the precipitate, and the resultant mixture was allowed to react at 37°C for 24 hours. After the reaction, 20 μl of a phosphate buffer solution containing 20 mg/ml Pronase E (code: P5147, Sigma Chemical Co.) was further added, and the resultant mixture was allowed to react at 37°C for 48 hours. After the reaction, 2 μl of 5.9 mg/ml Leucine aminopeptidase (code: L5006, Sigma Chemical Co.) and 10 μl of a phosphate buffer solution containing 1mg/ml Prolidase (code: P6675, Sigma Chemical Co.) were further added, and the resultant mixture was allowed to react at 37°C for 72 hours. The thus-obtained reaction solution was subjected to centrifugal ultrafiltration using an ultrafilter membrane with a molecular weight (MW) cut-off of 5,000 (Millipore Corporation), and the filtrate was collected.

The filtrate was applied to LC/MS analysis under the analysis conditions shown in Table 1 below. In the measurement according to the LC/MS method, identification and quantification of CMA were based on calculation from the ratio of the molecule ion peaks 233.2 and 70.0. From the measured values, the CMA concentration in the blood (nmol/ml) of each subject and the average concentrations in the respective groups were calculated.

[Table 1]

(Analysis Conditions)

(1) Analyzer:
TSQ 7000 triple-stage quadrupole mass spectrometer (Thermoquest, U.S.A.)

(2) Column
C-18M column (4.6 x 150 mm, Column Engineering, U.S.A.)

(3) Mobile phase
A: water containing 5 mmol/l nonafluoropentanoic acid
B: acetonitrile containing 5 mmol/l nonafluoropentanoic acid

(4) LC conditions
Reverse-phase chromatography
0.4 ml/min, Liner Gradient

(5) MS conditions
Electrospray ionization method (ESI-positive)
Ionizing energy, spray current and voltage: 72 eV, 1.5 mA and 4.5 kV
Collision gas: Argon, 2.0 mTorr, collision offset -25 V

(6) Detection conditions
LC retention time: 23 minutes

(continued)

(Analysis Conditions)
ESI/SIM (SIM: selected ion monitoring mode)
Quantifier transition (m/z)
Base peak at m/z 233, fragments m/z 70 & m/z 116

The measurement results regarding the CMA concentration in the blood of the high blood glucose group are shown in Table 2 below, and the measurement results regarding the CMA concentration in the blood of the healthy subject group are shown in Table 3 below. As shown in the following Table 2, the average CMA concentration in the blood of the high blood glucose group was 31.3 nmol/ml before the intake and 28.3 nmol/ml after the intake, and the difference between before and after the intake was -3.1 nmol/ml. In contrast, as shown in the following Table 3, the average CMA concentration in the blood of the healthy subject group was 27.2 nmol/ml before the intake and 26.4 nmol/ml after the intake, and the difference between before and after the intake was -0.8 nmol/ml. Thus, in both the high blood glucose group and the healthy subject group, the CMA concentration in the blood was decreased by the intake of the mixed extract. Furthermore, the decrease in the CMA concentration in the blood of the high blood glucose group was greater than that of the healthy subject group.
[Table 2]

| High blood glucose group | | | |
|---|---|---|---|
| No. | CMA concentration in blood (nmol/ml) | | Difference |
| | Before intake | After intake | |
| Average value | 31.3 | 28.3 | -3.1 |
| Standard deviation | 2.0 | 1.4 | 1.4 |

[Table 3]

| Healthy subject group | | | |
|---|---|---|---|
| No. | CMA concentration in blood (nmol/ml) | | Difference |
| | Before intake | After intake | |
| Average value | 27.2 | 26.4 | -0.8 |
| Standard deviation | 2.5 | 2.4 | 0.9 |

As described above, it was demonstrated that the CMA concentration in the blood is decreased by the intake of the mixed extract containing the extract of *Anthemis nobilis L.,* the extract of *Houttuynia cordata Thunberg,* the extract of *Crataegus oxyacantha L.,* and the extract of *Vitis vinifera L.* It was thus confirmed that the mixed extract is effective in inhibiting the decrease in elasticity of *in vivo* connective tissues and the progress of senescence of tissues containing a large amount of collagen, such as connective tissues.

Industrial Applicability

According to the present invention, by containing the above-described extract(s), it is possible to inhibit at least one of the following reaction pathways included in the CMA-producing pathway: the reaction pathway of glycation of a protein and the reaction pathway of oxidation of collagen by glyoxal. Thus, the present invention can inhibit the production of CMA and the denaturation of collagen, thereby inhibiting the decrease in elasticity of *in vivo* connective tissues and the progress of senescence of tissues containing a large amount of collagen, such as connective tissues. The present invention provides excellent safety because it uses plant extracts that have been used since ancient times. Also, the present invention provides excellent productivity because the plant extracts can be obtained in large quantities. The present invention is applicable to a wide range of fields such as industrial products, foods, and pharmaceuticals.

**Claims**

1. A carboxymethylarginine production inhibitor comprising:

   an extract of at least one plant selected from the group consisting of plants of *Compositae Anthemis, Saururaceae Houttuynia, Rosaceae Crataegus,* and *Vitaceae Vitis.*

2. The carboxymethylarginine production inhibitor according to claim 1 or 2, comprising:

   an extract of a plant of *Compositae Anthemis*;
   an extract of a plant of *Saururaceae Houttuynia*;
   an extract of a plant of *Rosaceae Crataegus*; and
   an extract of a plant of *Vitaceae Vitis.*

3. The carboxymethylarginine production inhibitor according to claim 1 or 2, wherein
   the plant of *Compositae Anthemis* is *Anthemis nobilis L.,*
   the plant of *Saururaceae Houttuynia* is *Houttuynia cordata Thunberg,*
   the plant of *Rosaceae Crataegus* is *Crataegus oxyacantha L.,* and
   the plant of *Vitaceae Vitis* is *Vitis vinifera L.*

4. A collagen denaturation inhibitor comprising:

   an extract of at least one plant selected from the group consisting of plants of *Compositae Anthemis, Saururaceae Houttuynia, Rosaceae Crataegus,* and *Vitaceae Vitis.*

5. The collagen denaturation inhibitor according to claim 4 comprising:

   an extract of a plant of *Compositae Anthemis*;
   an extract of a plant of *Saururaceae Houttuynia*;
   an extract of a plant of *Rosaceae Crataegus*; and
   an extract of a plant of *Vitaceae Vitis.*

6. The collagen denaturation inhibitor according to claim 4 or 5, wherein
   the plant of *Compositae Anthemis* is *Anthemis nobilis L.,*
   the plant of *Saururaceae Houttuynia* is *Houttuynia cordata Thunberg,*
   the plant of *Rosaceae Crataegus* is *Crataegus oxyacantha L.,* and
   the plant of *Vitaceae Vitis* is *Vitis vinifera L.*

7. A method for inhibiting production of carboxymethylarginine in collagen, the method comprising:

   adding the carboxymethylarginine production inhibitor according to any one of claims 1 to 3 to a sample containing collagen.

8. A method for inhibiting denaturation of collagen, the method comprising:

   adding the collagen denaturation inhibitor according to any one of claims 4 to 6 to a sample containing collagen.

9. A method for treating damage due to disease, senescence and hypofunction of tissue, and oxidative stress caused by production of carboxymethylarginine, the method comprising:

   administering the carboxymethylarginine production inhibitor according to any one of claims 1 to 3 to a living organism.

10. A method for treating damage due to disease, senescence and hypofunction of tissue, and oxidative stress caused by denaturation of collagen, the method comprising:

    administering the collagen denaturation inhibitor according to any one of claims 4 to 6 to a living organism.

Extract of *Anthemis nobilis L.*

FIG. 1A

Extract of *Houttuynia cordata Thunberg*

FIG. 1B

Extract of *Crataegus oxyacantha L.*

FIG. 1C

Extract of *Vitis vinifera L.*

FIG. 1D

Mixed extract

FIG. 1E

Extract of *Anthemis nobilis L.*

FIG. 2A

Extract of *Houttuynia cordata Thunberg*

FIG. 2B

Extract of *Crataegus oxyacantha L.*

FIG. 2C

Extract of *Vitis vinifera L.*

FIG. 2D

Mixed extract

FIG. 2E

Extract of *Anthemis nobilis L.*

FIG. 3A

Extract of *Houttuynia cordata Thunberg*

FIG. 3B

Extract of *Crataegus oxyacantha L.*

FIG. 3C

Extract of *Vitis vinifera L.*

FIG. 3D

<table>
<tr><td colspan="2" align="center">**INTERNATIONAL SEARCH REPORT**</td><td>International application No.<br>PCT/JP2010/061063</td></tr>
</table>

A. CLASSIFICATION OF SUBJECT MATTER
*A61K36/73*(2006.01)i, *A61P3/10*(2006.01)i, *A61P9/10*(2006.01)i, *A61P19/00*(2006.01)i, *A61P19/10*(2006.01)i, *A61P25/28*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K36/73, A61P3/10, A61P9/10, A61P19/00, A61P19/10, A61P25/28, A61P43/00

Documentation searched other than minimum documentation to the extent that such documents are included in the fields searched
Jitsuyo Shinan Koho          1922-1996     Jitsuyo Shinan Toroku Koho     1996-2010
Kokai Jitsuyo Shinan Koho    1971-2010     Toroku Jitsuyo Shinan Koho     1994-2010

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2005-035911 A  (Arkray, Inc.),<br>10 February 2005 (10.02.2005),<br>paragraphs [0002], [0004], [0037] to [0054]<br>(Family: none) | 1-8<br>1-8 |
| X<br>Y | JP 2008-231031 A  (Arkray, Inc.),<br>02 October 2008 (02.10.2008),<br>paragraphs [0003], [0025] to [0030]<br>& WO 2008/0114536 A1     & US 2010/009016 A1 | 1-8<br>1-8 |
| Y | JP 2002-243732 A  (Nippi, Inc.),<br>28 August 2002 (28.08.2002),<br>paragraphs [0002], [0004]<br>(Family: none) | 1-8 |

☐ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>27 July, 2010 (27.07.10) | Date of mailing of the international search report<br>03 August, 2010 (03.08.10) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2010/061063 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 9, 10
   because they relate to subject matter not required to be searched by this Authority, namely:
   Claims 9 and 10 involve "methods for treatment of the human body or animal body by surgery or therapy".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3889190 B
- JP 2009096731 A
- JP 2002243732 A

**Non-patent literature cited in the description**

- **Iijima et al.** *Biochem. J.,* 2000, vol. 347, 23-27
- **Odani et al.** *Biochem. Biophys. Res. Commun.,* 2001, vol. 285, 1232-1236
- **Thornalley.** *Ann. N.Y. Acad. Sci.,* 2005, vol. 1043, 111-117